# EUROPEAN PATENT APPLICATION

(11) **EP 4 386 679 A1**
(43) Date of publication of application: **19.06.2024**
(21) Application number: 22213496.7
(22) Date of filing: 14.12.2022
(51) Int. Cl.: G06T 11/00

(54) **FAST VIBRATION CORRECTION FOR IMAGES WITH METAL OBJECTS IN CBCT**

(71) Applicant: Koninklijke Philips N.V., 5656 AG Eindhoven (NL)
(72) Inventor: BRENDEL, Bernhard Johannes, Eindhoven (NL); SCHÄFER, Dirk, Eindhoven (NL)
(74) Representative: Philips Intellectual Property & Standards

(57) **Abstract**

The present invention relates to vibration correction. A vibration artifact suppression method (100) is provided that comprises the following steps: a) reconstructing (110) a three-dimensional image of an object of interest based on projection data acquired with an X-ray imaging system, b) determining (120) whether the reconstructed three-dimensional image contains a metal object, and c) performing one of the following steps based on a result of the determination: c1) in response to determining that the reconstructed three-dimensional image contains a metal object, performing (130) a vibration correction method based on the metal object segmented in the reconstructed three-dimensional image; or c2) in response to determining that the reconstructed three-dimensional image contains no metal object, performing (140) a vibration correction method based on the object of interest segmented in the reconstructed three-dimensional image. With the vibration artifact suppression method, the number of iterations may be strongly reduced, leading to a significant speed up of the overall reconstruction time.

## Description

### FIELD OF THE INVENTION

The present invention relates to a vibration artifact suppression method, to a vibration artifact suppression device, to an X-ray imaging system, to a computer program product, and to a computer-readable medium.

### BACKGROUND OF THE INVENTION

Three-dimensional (3D) imaging with interventional C-arm systems is a field of increasing interest for numerous interventional procedures. For data acquisition, a motorized movement of the C-arc is performed to move tube and detector around the region of interest. During this movement, projection data is acquired that can be used along with knowledge about the position of tube and detector to reconstruct a 3D image.

In the geometry data derived from the calibration procedure, it can be observed that the C-arc vibrates during the acquisition due to acceleration forces linked to the movement. If phase and/or amplitude of the vibration patterns deviate between calibration procedure and interventional data acquisition, severe image artifacts may arise that impair image quality considerably. This may in particular affect mobile C-arm systems as their vibration is considerably larger than for fixed systems.

### SUMMARY OF THE INVENTION

There may, therefore, be a need to improve vibration correction, e.g., for an X-ray imaging system, such as a mobile C-arm system.

The object of the present invention is solved by the subject-matter of the appended independent claims, wherein further embodiments are incorporated in the dependent claims.

In a first aspect, there is provided a vibration artifact suppression method. The method comprises:
a) reconstructing a three-dimensional image of an object of interest based on projection data acquired with an X-ray imaging system;
b) determining whether the reconstructed three-dimensional image contains a metal object, and
c) performing a vibration correction method based on a structure of interest segmented in the reconstructed three-dimensional image, wherein the structure of interest is selected based on an outcome of the determining step.

In other words, the present disclosure proposes a vibration correction method for X-ray systems, e.g., for mobile C-arm systems, to effective reduce vibration artifacts.

In certain examples, the vibration artifact suppression method disclosed herein includes an initial reconstruction in which specific reconstructed image structures are segmented. The segmented image structures are forward projected onto the originally acquired projections and registered with the corresponding structures in the projection data to estimate the vibration. Using the estimated vibration information thus determined, a new reconstruction can be performed having reduced vibration artefacts. If necessary, the process may go through multiple iterations in order to obtain a sufficient image quality.

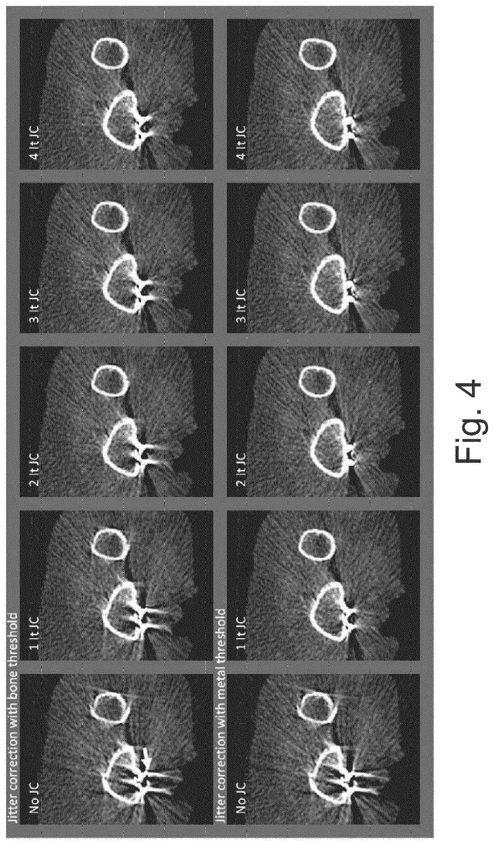

The image structures to be segmented are selected based on the outcome of determining the presence of a metal object. That is, in some cases in which the reconstructed image contains a metal object, the image structures to be segmented include metal objects and step c) comprises c1) performing the vibration correction method based on the segmented metal object(s). In this case, advantageously, the number of iterations can be low, also for mobile C-arm 3D imaging. This will be explained in detail hereinafter and in particular with respect to the images shown in FIG. 4. Therefore, in case one or more metal objects are present, the one or more metal objects are segmented and used as a basis for the vibration estimation.

In other cases, in which the reconstructed image does not contain any metal object, or only a limited amount of metal is present, for example bone structures are segmented instead of metal, and used as a basis for the vibration estimation. That is, step c) thus comprises c2) performing the vibration correction method based on the segmented bone structure(s).

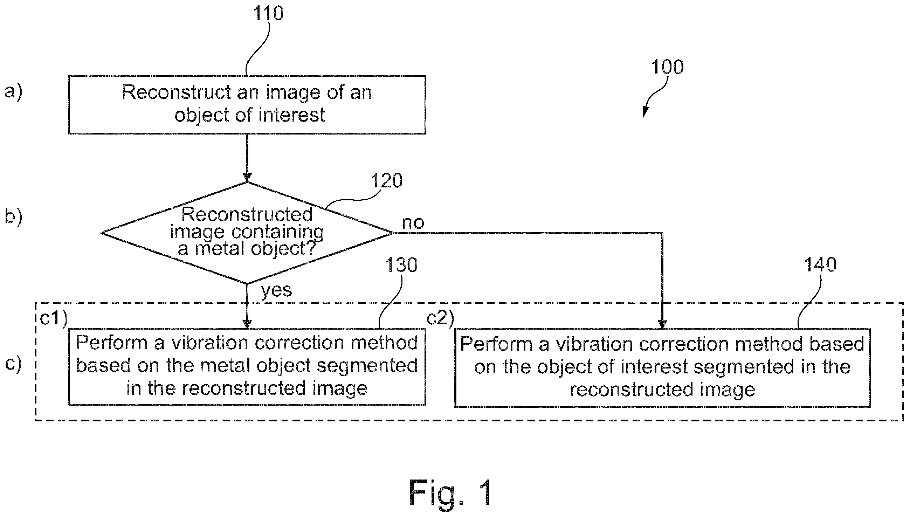

This will be explained in detail hereinafter and in particular with respect to the example shown in FIG. 1.

In an embodiment, step c1) further comprises determining an amount of the metal object in the reconstructed three-dimensional image and determining, based on the amount of the metal object, whether to perform the vibration correction method based on the metal object segmented in the reconstructed three-dimensional image.

Apart from the mere decision if an image contains metal or not, it is optional to check if enough metal structures are available to robustly estimate the vibration. This may be done by checking how many image slices comprise a significant part of the metal structures. Possible other criteria can be the volume percentage of the metal or the volume percentage of the convex hull of the metal, since the distribution within the field-of-view (FOV) is an important aspect for a robust vibration correction.

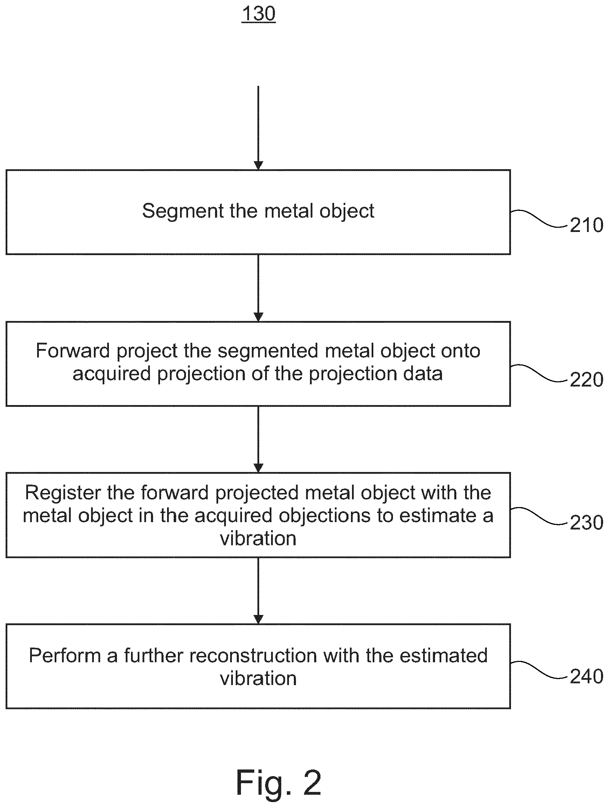

In an embodiment, the amount of the metal object is determined based on at least one of:
- a number of image slices in the projection data that comprise the metal object;
- a volume percentage of the metal object; or
- a volume percentage of a convex hull of the metal object.
In an embodiment, the vibration correction method comprises:
- segmenting the metal object in the reconstructed three-dimensional image;
- forward projecting the segmented metal object onto acquired projections of the projection data;
- registering the forward projected metal object with the metal object in the acquired projections to estimate a vibration of the C-arm X-ray imaging system during acquisition of the projection data; and
- performing a further reconstruction with the estimated vibration.
This will be explained in detail hereinafter and in particular with respect to the example shown in FIG. 2. According to an embodiment, the vibration correction method in step c2) comprises:
- segmenting the bone structure in the reconstructed three-dimensional image;
- forward projecting the segmented bone structure onto acquired projections of the projection data;
- registering the forward projected bone structure with a corresponding bone structure in the acquired projections to estimate a vibration of the C-arm X-ray imaging system during acquiring the projection data; and
- performing a further reconstruction with the estimated vibration.

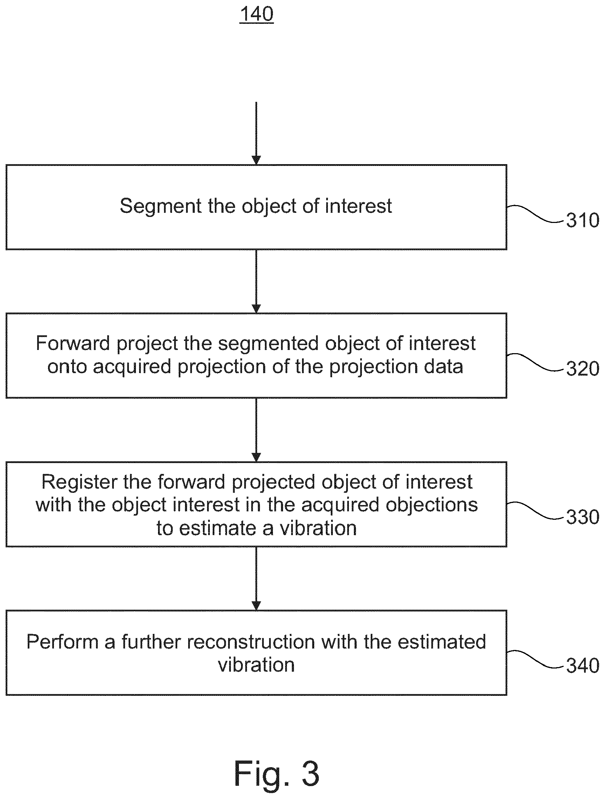

This will be explained in detail hereinafter and in particular with respect to the example shown in FIG. 3.

In certain examples, the forward projections and reconstructions are iteratively performed.

In certain examples, the segmented metal object or the segmented bone structure is forward projected on to acquired projections with a reduced resolution.

The computational time for iterative image reconstruction is often dominated by the forward-projection operations and back-projection operations. By using acquired projections with a reduced resolution, the computation time may be greatly reduced.

In an embodiment, in step a) the three-dimensional image of the object of interest is reconstructed with a lower resolution than a final image reconstruction.

The initial reconstruction and all reconstructions within the vibration correction iterations may be performed with lower resolution than the final image reconstruction to save computation time.

In a second aspect, there is provided a vibration artifact suppression device comprising a processor configured to perform the steps of the method of any one of the preceding claims.

In a third aspect, there is provided an X-ray imaging system, comprising:
- a C-arm X-ray imaging system configured to acquire projection data of an object of interest; and a vibration artifact suppression device according to the second aspect and any associated example.

In certain embodiments, the C-arm X-ray imaging system is a mobile C-arm X-ray imaging system, for example used in medical imaging.

In another aspect, there is provided a computer program product comprising instructions which, when the program is executed by a processor, cause the processor to carry out the steps of the method of the third aspect and any associated example.

In a further aspect of the present invention, there is provided a computer-readable storage medium having stored thereon the computer program product.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
FIG. 1 illustrates a flow diagram describing an exemplary vibration artifact suppression method.
FIG. 2 illustrates a flow diagram of a step to perform a vibration correction method based on the metal object segmented in the reconstructed image, according to one implementation.
FIG. 3 illustrates a flow diagram of a step to perform a vibration correction method based on the object of interest segmented in the reconstructed image, according to one implementation.
FIG. 4 illustrates an image processed with different numbers of vibration correction iterations for bone structures and metallic foreign bodies.
FIG. 5 illustrates an exemplary X-ray imaging system.

### DETAILED DESCRIPTION OF EMBODIMENTS

3D imaging with interventional C-arm systems is a field of increasing interest for numerous interventional procedures. For data acquisition, a motorized movement of the C-arc is performed to move tube and detector around the region of interest. During this movement, projection data is acquired that can be used along with knowledge about the position of tube and detector to reconstruct a 3D image. The knowledge about the position of tube and detector is obtained from a geometry calibration procedure, in which the data acquisition is performed with a precisely known geometric object in the region of interest.

In the geometry data derived from the calibration procedure, it can be observed that the C-arc vibrates during the acquisition due to acceleration forces linked to the movement. Furthermore, from repeated calibrations, it can be seen that this vibration varies in amplitude and phase for different acquisition runs. If phase and/or amplitude of the vibration patterns deviate between calibration procedure and interventional data acquisition, severe image artifacts may arise that impair image quality considerably (see FIG. 4, top row, left image).

To reduce these artifacts, a vibration correction (VC) method has been developed. The method starts with an initial reconstruction. In the initial reconstruction, certain image structures are segmented, and forward projected onto the acquired projections. Then, the forward projected structures are registered with the corresponding structures in the acquired original projections to estimate the vibration. With the estimated vibration a new reconstruction resulting in reduced vibration artifacts is performed.

The vibrations in mobile C-arm systems may be particularly large, considerably larger than for fixed systems, so that the processing may have to be repeated iteratively (see FIG. 4, top row). That is, in an iterative process, the vibration correction reconstruction is repeatedly used to segment and forward project bone structures that are in turn used to refine the vibration estimate in another registration step. In the illustrated example (FIG. 4, top row) the processing is repeated 4 times to achieve an acceptable level of artifact suppression. Thus, five image reconstructions, four forward projections, and four registrations have to be performed, leading to high computation times for reconstruction.

It has been found that, for images which contain metal objects, the number of iterations of the vibration correction can be considerably reduced if the metal objects are segmented, forward projected, and used for registration. This is especially of interest, since for these datasets on top of the vibration correction a metal artifact reduction (MAR) may be performed, which is also computationally very demanding. With the vibration artifact suppression method as disclosed herein, the number of iterations can be strongly reduced, leading to a significant speed up of the overall reconstruction time.

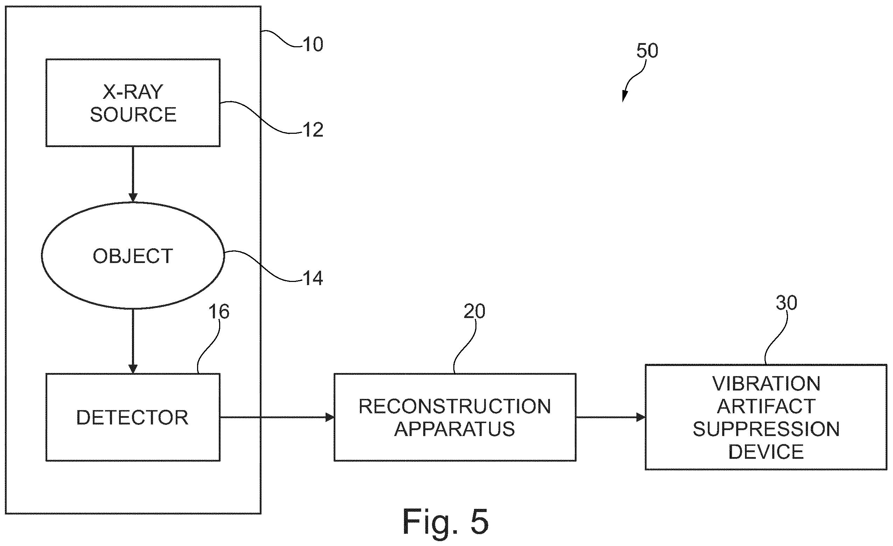

FIG. 1 illustrates a flow diagram describing an exemplary vibration artifact suppression method 100 according to an embodiment of the present disclosure. The vibration artefact suppression method 100 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the exemplary method may be implemented as the apparatus 30 shown in FIG. 5.

In step 110, i.e., step a), the vibration artifact suppression method 100 comprises a step of reconstructing a 3D image of an object of interest based on projection data acquired with an X-ray imaging system, e.g., a mobile C-arm system. The reconstructed image may also be referred to as initial reconstructed image or initial image. In some examples, the projection data may be obtained by performing a scan using the X-ray imaging system. In some other examples, the projection may be obtained by recalling from computer memory projection data that has been previously obtained. The obtained projection data may be at the native resolution of the X-ray detectors of the X-ray imaging system.

In some examples, the initial image may be generated using down-sampled projection data and using an image resolution for the initial image that is commensurate with the down-sampled projection data. The down-sampling of the projection data may be achieved using any known down-sampling method. In some examples, the fine-resolution pixels may be grouped into pixel groups corresponding to the coarse-resolution pixels of the down-sampled data, and then the values of the respective pixel groups of the fine-resolution pixels are averaged or summed to generate the values of the coarse-resolution pixels. In some examples, the fine-resolution pixels can be resampled onto a grid or other pixel pattern for the coarse-resolution pixels using interpolation, extrapolation, and/or integration from the fine-resolution grid onto the coarse-resolution grid.

The initial reconstructed image may be generated using any known reconstruction method. Examples of the reconstruction method for reconstructing the 3D image of the object of interest may include, but are not limited to, filtered back-projection (FBP), a Feldkamp-Davis-Kress (FDK) reconstruction method, an IR method using e.g., an objective function with a least-squares or a penalized-weighted-least-square data -fidelity term, and a regularization term.

In step 120, i.e., step b), the vibration artifact suppression method 100 comprises a step of determining whether the reconstructed three-dimensional image contains a metal object. The metal object may also be referred to as metallic foreign body. The decision if the initial image contains a metal object or not can be taken with different means.

In some examples, if the image values are quantitatively correct Hounsfield unit (HU) values, the decision if the initial image contains metal or not may be made based on the number of voxel values that exceed a defined threshold. This is because the HU values demonstrate significant difference between anatomical structures and metallic foreign bodies. For example, the HU value for bones may range from +300 to +1900. Metallic foreign bodies typically have a higher HU value. For example, copper has +14000 HU, silver has +17000 HU, steel has +20000 HU, and gold has +30000 HU. Therefore, an HU threshold may be defined to distinguish the metallic foreign body from the anatomical structures.

In some examples, if the image values are not quantitatively correct HU values, the decision if the initial image contains metal or not may be made based on the histogram of the initial image. An image histogram is a gray-scale value distribution showing the frequency of occurrence of each gray-level value. The histogram analysis is based on an assumption that the gray-scale values of the anatomical structures and the metal object are distinguishable. This may result in two peaks appearing on a histogram. Those peaks usually overlap, yet a minimum in between can be detected in order to separate both objects e.g., using a linear support vector machine. It will be appreciated that the two objects may be separated using other AI-based (e.g., neural networks) or non-AI-based methods based on the histogram of the initial image, or the image itself.

In some examples, the decision if the initial image contains metal or not may be made based on a user input, e.g., via a graphical user interface (GUI).

Optionally, step 120 may further comprise a step of determining an amount of the metal object in the reconstructed three-dimensional image and determining, based on the amount of the metal object, whether to perform the vibration correction method based on the metal object segmented in the reconstructed three-dimensional image. In other words, apart from the mere decision if an image contains metal or not, it may be checked if enough metal structures are available to robustly estimate the vibration.

In some examples, the amount of the metal object may be determined based on a number of image slices in the projection data that comprise the metal object. For example, the step 120 may further comprise checking how many image slices comprise a significant part of the metal structures. A threshold may be set for the number of image slices. If the number of image slices that comprises a significant part of the metal structures is equal to or greater than this threshold, it may be determined to perform the vibration correction method based on the metal object segmented in the reconstructed three-dimensional image.

In some examples, the amount of the metal object may be determined based on a volume percentage of the metal object and/or a volume percentage of a convex hull of the metal object, since the distribution within the field-of-view (FOV) is an important aspect for a robust vibration correction.

If it is determined that the reconstructed three-dimensional image contains a metal object, the vibration artifact suppression method 100 proceeds from step 120 to step 130, i.e., step c1), and a vibration correction method is performed based on the metal object segmented in the reconstructed image. If there is no metal object present, or the amount of metal is below a threshold for example, the vibration artifact suppression method 100 proceeds from step 120 to step 140, i.e., step c2), and a vibration correction method is performed based on other image structures (e.g. bony structures) segmented in the reconstructed image.

FIG. 2 illustrates a flow diagram describing one implementation of step 130.

In step 210 of step 130, the metal object in the reconstructed 3D image is segmented. In some examples, simple thresholding with either a fixed threshold for images with correct HU values or with an estimated threshold, e.g., based on the histogram of the image, may be used to segment the metal object. In some examples, AI-based segmentation of the metal object, e.g., based on U-Nets, may be implemented in some examples.

In step 220 of step 130, once the metal object is segmented, the segmented metal object is forward projected onto the acquired projections. In some examples, the segmented metal object may forward projected on to acquired projections with a reduced resolution, e.g., onto a low-resolution grid.

In step 230 of step 130, the forward projected metal object is registered with the metal object in the acquired projections to estimate a vibration of the X-ray imaging system during acquiring the projection data. This may be done by standard means as already available for the vibration correction based on e.g., bone structures.

In step 240 of step 130, a further reconstruction is performed with the estimated vibration resulting in reduced vibration artifacts.

To improve the situation, steps 210 to 240 may be repeated iteratively.

FIG. 3 illustrates a flow diagram describing one implementation of step 140.

In step 310 of step 140, the object of interest (e.g., bone structures) in the reconstructed 3D image is segmented. In some examples, simple thresholding, with either a fixed threshold (for images with correct HU values) or with an estimated threshold, e.g., based on the histogram of the image may be used to segment structures of interest for performing the vibration correction, such as bone structures. In some examples, AI-based segmentation of the structures of interest, e.g., based on U-Nets, may be performed.

In step 320 of step 140, once the relevant structure(s) has been segmented, the segmented structure is forward projected onto the acquired projections. In some examples, the segmented structure may be forward projected on to acquired projections with a reduced resolution, e.g., onto a low-resolution grid.

In step 330 of step 140, the forward projected structure is registered with the corresponding structure in the acquired original projections to estimate a vibration of the X-ray imaging system during acquiring the projection data.

In step 340 of step 140, a further reconstruction is performed with the estimated vibration resulting in reduced vibration artifacts.

To improve the situation, steps 310 to 340 may be repeated iteratively.

FIG. 4 illustrates an image processed with different numbers of vibration correction iterations (from left to right) for bone structures and metallic foreign bodies. The image shown in FIG. 4 comprises metallic foreign bodies. The top row of FIG. 4 shows vibration correction that is only based on bone structures, while the bottom row of FIG. 4 shows vibration correction based on metal structures. As can be seen from FIG. 4, vibration artifacts, such as the one denoted by the white arrow in the top left image, are suppressed much faster if vibration correction is based on metal structures.

FIG. 5 shows an exemplary X-ray imaging system 50 according to some embodiments of the present disclosure. Examples of the X-ray imaging system may include, but are not limited to, a C-arm system, a computed tomography (CT) system, a digital X-ray radiography (DXR) system, and an image-guided therapy (IGT) system. In some examples, the C-arm system may be a mobile C-arm system. The following discussion of the X-ray system 50 is merely an example of such implementation and is not intended to be limiting in terms of modality.

The X-ray imaging system 50 comprises an image acquisition apparatus 10, a reconstruction apparatus 20, and a vibration artifact suppression device 30.

The image acquisition apparatus 10 comprises an X-ray detector 16 opposing an X-ray source 12. The image acquisition apparatus 10 is configured to scan an object of interest 14 to generate projection data, namely raw-image, comprising one or more image slices, each representative of a certain thickness of the object being scanned in a certain angle of projection.

The reconstruction apparatus 20 is configured to receive projection data which may be obtained by performing a scan using the X-ray system 10, and to determine an initial reconstructed image. The initial reconstructed image may be generated using any known reconstruction method. Examples of the reconstruction method for reconstructing the 3D image of the object of interest may include, but are not limited to, filtered back-projection (FBP), a Feldkamp-Davis-Kress (FDK) reconstruction method, an IR method using e.g., an objective function with a least-squares or a penalized-weighted-least-square data-fidelity term, and a regularization term. The reconstruction apparatus 20 then provides the initial reconstructed image to the vibration artifact suppression device 30.

The vibration artifact suppression device 30 is configured to perform the vibration artifact suppression method as disclosed herein, such as the method shown in FIG. 1, to obtain a reconstructed image with reduced vibration artifacts.

In general, the vibration artifact suppression device 30 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although FIG. 5 may show a limited number of components by way of example, it can be appreciated that a greater or a fewer number of components may be employed for a given implementation.

In some implementations, the vibration artifact suppression device 30 may be embodied as, or in, a device or apparatus, such as a server, workstation, or mobile device. The apparatus 10 may comprise one or more microprocessors or computer processors, which execute appropriate software. For example, the vibration artifact suppression device 30 may have a processing unit which may be embodied by one or more of these processors. The software may have been downloaded and/or stored in a corresponding memory, e.g., a volatile memory such as RAM or a non-volatile memory such as flash. The software may comprise instructions configuring the one or more processors to perform the functions as described herein.

It is noted that the vibration artifact suppression device 30 may be implemented with or without employing a processor, and also may be implemented as a combination of dedicated hardware to perform some functions and a processor (e.g., one or more programmed microprocessors and associated circuitry) to perform other functions. For example, the vibration artifact suppression device 30 may be implemented in the device or apparatus in the form of programmable logic, e.g., as a Field-Programmable Gate Array (FPGA). In general, each functional unit of the apparatus may be implemented in the form of a circuit.

In some implementations, the vibration artifact suppression device 30 may also be implemented in a distributed manner. For example, some or all units of the apparatus 10 may be arranged as separate modules in a distributed architecture and connected in a suitable communication network, such as a 3rd Generation Partnership Project (3GPP) network, a Long Term Evolution (LTE) network, Internet, LAN (Local Area Network), Wireless LAN (Local Area Network), WAN (Wide Area Network), and the like.

Although FIG. 5 may show the reconstruction apparatus 20 and the vibration artefact suppression device 30 as two separate devices by way of example, in some other examples, the vibration artifact suppression device 30 may reside in the reconstruction apparatus 20, e.g., running as a software. In another exemplary embodiment of the present invention, a computer program or a computer program element is provided that is characterized by being adapted to execute the method steps of the method according to one of the preceding sfs45kk, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A vibration artifact suppression method (100), comprising:
a) reconstructing (110) a three-dimensional image of an object of interest based on projection data acquired with an X-ray imaging system;
b) determining (120) whether the reconstructed three-dimensional image contains a metal object, and
c) performing (130, 140) a vibration correction method based on a structure of interest segmented in the reconstructed three-dimensional image, wherein the structure of interest is selected based on an outcome of the determining step.

2. The vibration artifact suppression method according to claim 1, wherein the structure of interest is the metal object and step c) comprises:
c1) performing (130) the vibration correction method based on the metal object.

3. The vibration artifact suppression method according to claim 2,
wherein step c1) further comprises determining an amount of the metal object in the reconstructed three-dimensional image and determining, based on the amount of the metal object, whether to perform the vibration correction method based on the metal object segmented in the reconstructed three-dimensional image.

4. The vibration artifact suppression method according to claim 3,
wherein the amount of the metal object is determined based on at least one of:
- a number of image slices in the projection data that comprise the metal object;
- a volume percentage of the metal object; or
- a volume percentage of a convex hull of the metal object.

5. The vibration artifact suppression method according to any one of the preceding claims 2 to 4,
wherein the vibration correction method in step c1) comprises:
- segmenting (210) the metal object in the reconstructed three-dimensional image;
- forward projecting (220) the segmented metal object onto acquired projections of the projection data;
- registering (230) the forward projected metal object with the metal object in the acquired objections to estimate a vibration of the X-ray imaging system during acquiring the projection data; and
- performing (240) a further reconstruction with the estimated vibration.

6. The vibration artifact suppression method according to claim 1, wherein the structure of interest is a bone structure and step c) comprises:
c2) performing (140) the vibration correction method based on the bone structure segmented in the reconstructed three-dimensional image.

7. The vibration artifact suppression method according to claim 6,
wherein the vibration correction method in step c2) comprises:
- segmenting (310) the bone structure in the reconstructed three-dimensional image;
- forward projecting (320) the segmented bone structure onto acquired projections of the projection data;
- registering (330) the forward projected bone structure with a corresponding bone structure in the acquired projections to estimate a vibration of the X-ray imaging system during acquiring the projection data; and
- performing a further reconstruction with the estimated vibration.

8. The vibration artifact suppression method according to claim 5 or 7,
wherein the forward projections and reconstructions are iteratively performed.

9. The vibration artifact suppression method according to any one of claims 5 to 8,
wherein the segmented metal object or the bone structure is forward projected on to acquired projections with a reduced resolution.

10. The vibration artifact suppression method according to any one of the preceding claims,
wherein in step a) the three-dimensional image of the object of interest is reconstructed with a lower resolution than a final image reconstruction.

11. A vibration artifact suppression device (30) comprising a processor configured to perform the steps of the method of any one of the preceding claims.

12. An X-ray imaging system (50), comprising:
- an X-ray imaging system configured to acquire projection data of an object of interest; and
- a vibration artifact suppression device according to claim 11.

13. The X-ray imaging system according to claim 12,
wherein the X-ray imaging system is a mobile C-arm X-ray imaging system.

14. A computer program product comprising instructions which, when the program is executed by a processor, cause the processor to carry out the steps of the method of any one of claims 1 to 10.

15. A computer-readable storage medium having stored thereon the computer program product of claim 14.
